# EUROPÄISCHE PATENTANMELDUNG

(11) **EP 4 369 320 A1**
(43) Veröffentlichungstag der Anmeldung: **15.05.2024**
(21) Anmeldenummer: 23208349.3
(22) Anmeldetag: 07.11.2023
(51) Int. Cl.: G08B 21/22

(54) **VORRICHTUNG ZUM ERFASSEN EINER BEWEGUNG EINER PERSON**

(30) Priorität: 11.11.2022 DE 202022106356 U
(71) Anmelder: DewertOkin GmbH, 32278 Kirchlengern (DE)
(72) Erfinder: HILLE, Armin, 33659 Bielefeld (DE)
(74) Vertreter: Kleine, Hubertus

(57) **Zusammenfassung**

Die Erfindung betrifft eine Vorrichtung zum Erfassen einer Bewegung einer Person im Bereich eines Seitenteils (4) eines Betts (1), insbesondere eines Pflege- oder Klinikbetts. Die Vorrichtung zeichnet sich dadurch aus, dass am Bett (1) oberhalb des Seitenteils (4) ein Sensor (11) angeordnet ist, der eine Entfernung zu einem Gegenstand oder Körperteil misst, der oder das sich entlang einer Linie oberhalb des Seitenteils (4) befindet.

## Beschreibung

Die Erfindung betrifft eine Vorrichtung zum Erfassen einer Bewegung einer Person im Bereich eines Seitenteils eines Betts, insbesondere eines Pflege- oder Klinikbetts.

Um zu verhindern, dass Personen, insbesondere kranke oder pflegebedürftige Personen, ein Bett über ein anmontiertes Seitenteil, z.B. ein Seitengitter, verlassen, sind verschiedenen Überwachungs- und Detektionsvorrichtungen bekannt. Solche Vorrichtung geben in der Regel entweder unmittelbar oder über eine Kommunikationsverbindung ein Warn- oder Alarmsignal aus, durch das Pflege- bzw. Betreuungspersonal informiert wird und so verhindern kann, dass sich die Person eventuell bei einem Überklettern des Seitenteils verletzt.

In der Schrift DE 197 32 705 A1 ist beispielsweise eine Überwachungseinrichtung gezeigt, bei der eine Lichtschranke mit einem Sender und Empfänger an einem und Reflektor an einem gegenüberliegenden Kopf- bzw. Fußteil des Betts angeordnet ist. Diese Anordnung erfordert jedoch eine genaue Ausrichtung von Sender und Empfänger bzw. Reflektor, um den Bereich über dem Seitenteil überwachen zu können.

Aus der Schrift DE 10 2005 023 564 B4 ist bekannt, eine berührungslos arbeitende Detektionsvorrichtung an einem Kopfteil eines Betts anzuordnen, die einen Bereich über dem Seitenteil des Betts überwacht und eine Bewegung dadurch erkennt, dass eine ausgesendete elektromagnetische Welle reflektiert und ihre Reflexion wieder empfangen wird. Dabei ist der Erfassungsbereich des Detektors scharf begrenzt, so dass Bewegungen, die zum Beispiel in einem Bereich jenseits des Fußteils des Betts stattfinden, nicht zum Auslösen eines Fehlalarms führen.

Es ist eine Aufgabe der vorliegenden Erfindung, eine Vorrichtung der eingangs genannten Art zu schaffen, mit der ein mögliches Überklettern eines Seitenteils eines Betts ohne großen Justieraufwand sicher erkannt wird, wobei die Vorrichtung auch für nur abschnittsweise ausgebildete Seitenteile geeignet sein soll. Zudem soll die Vorrichtung die Möglichkeit bieten, mit wenig Zusatzaufwand zwischen zufälligen Bewegungen im Bereich des Seitenteils und einem tatsächlichen Überkletterversuch zu unterscheiden.

Diese Aufgabe wird gelöst durch eine Vorrichtung mit den Merkmalen des unabhängigen Anspruchs. Vorteilhafte Ausgestaltungen und Weiterbildungen sind Gegenstand der abhängigen Ansprüche.

Eine erfindungsgemäße Vorrichtung zum Erfassen einer Bewegung einer Person der eingangs genannten Art zeichnet sich dadurch aus, dass am Bett oberhalb des Seitenteils ein Sensor angeordnet ist, der eine Entfernung zu einem Gegenstand oder Körperteil misst, der oder das sich entlang einer Linie oberhalb des Seitenteils befindet. Reflexionen durch Gegenstände oder Körperteile, die in diesen Bereich eindringen, können mithilfe des Sensors erfasst werden. Eine erfasste Bewegung in diesem Bereich ist ein frühes Indiz dafür, dass die sich im Bett befindende Person versucht, das Bett durch Überklettern des Seitengitters zu verlassen.

Die Messung der Entfernung ermöglicht es, zwischen Bewegungen, die relevant im Hinblick auf ein mögliches Überklettern des Seitenteils sind, und solchen, die beispielsweise außerhalb des Betts liegen, zu unterscheiden. Weiter bietet die erfindungsgemäße Messung der Entfernung die zusätzliche Information, ob bei einem nur abschnittsweise ausgebildeten Seitenteil dieses tatsächlich überklettert wird oder das Bett zulässig in dem freien Bereich neben dem Seitengitter verlassen wird.

Schließlich ist die Montage gegenüber Systemen mit einer Lichtschranke vereinfacht, da nur der Sensor an einem Bettteil, bevorzugt an einem Kopfteil, anzubringen und auszurichten ist und kein zugeordneter Reflektor am gegenüber liegenden Bettteil benötigt wird.

In einer vorteilhaften Ausgestaltung der Vorrichtung ist der Sensor ein Lidar (Light detection and ranging)-Sensor oder auch ein Ultraschallsensor. Beide eigenen sich gut zur Entfernungsmessung in dem benötigten Abstandsbereich von einigen Zentimetern bis etwa 2 Metern. Alternativ ist auch eine lichtbasierte, beispielsweise infrarotlichtbasierte Triangulationsmessung in Reflexionsgeometrie denkbar, um den Abstand zum Reflexionsort zu messen.

Bevorzugt umfasst der Sensor einen Sender und einen Empfänger für ein Signal in Reflexionsgeometrie, insbesondere integral angeordnet und bereits geeignet zueinander ausgerichtet. Dadurch entfallen sensitive Ausrichtungen von Sender und Empfänger zueinander. Zur Entfernungsmessung kann eine Laufzeitmessung zwischen einem Senden und einem Empfangen des Signals vorgesehen ist.

In einer weiteren vorteilhaften Ausgestaltung der Vorrichtung ist der Sensor in einer Sensoranordnung angeordnet, die eine Ansteuerschaltung für den Sensor, eine Auswerteschaltung für den Sensor und/oder eine Kommunikationseinrichtung aufweist. Bei einer derartigen Vorrichtung sind alle benötigten Komponenten in einer Einheit integriert. Diese kann dann z.B. am Kopfteil positioniert werden, wobei zur Installation lediglich zum Beispiel eine Federklemme, Schraubklemme, Klettband, doppelseitiges Klebeband oder ähnliches benötigt wird. Insbesondere wenn die Kommunikationseinrichtung mit integriert ist und einen drahtlosen Kommunikationskanal nutzt, ist nur ein geringer oder gar kein Verdrahtungsaufwand erforderlich.

Die Erfindung wird nachfolgend anhand eines Ausführungsbeispiels mithilfe von Figuren näher erläutert.

Die Figuren zeigen:
- Figur 1: ein erstes Beispiel eines Betts mit einem Seitenteil und einer erfindungsgemäßen Vorrichtung; und
- Figur 2: ein zweites Beispiel eines Betts mit einem Seitenteil und einer erfindungsgemäßen Vorrichtung.

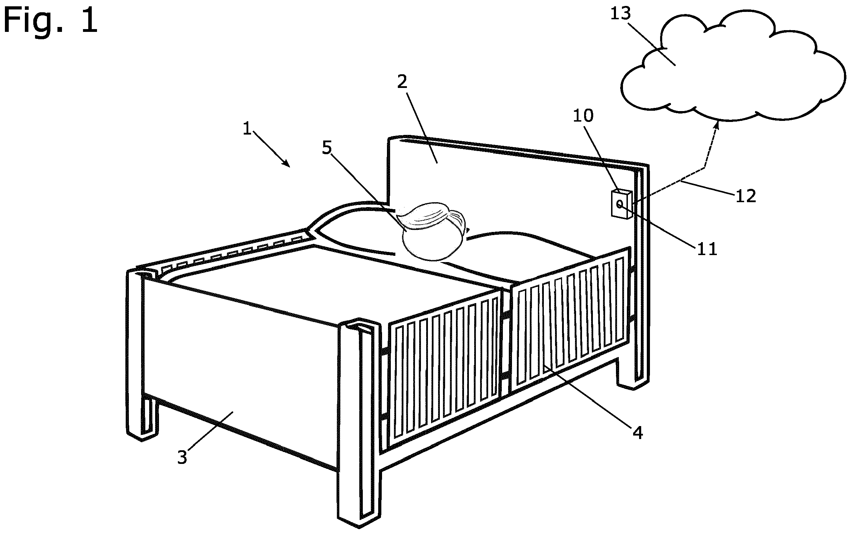

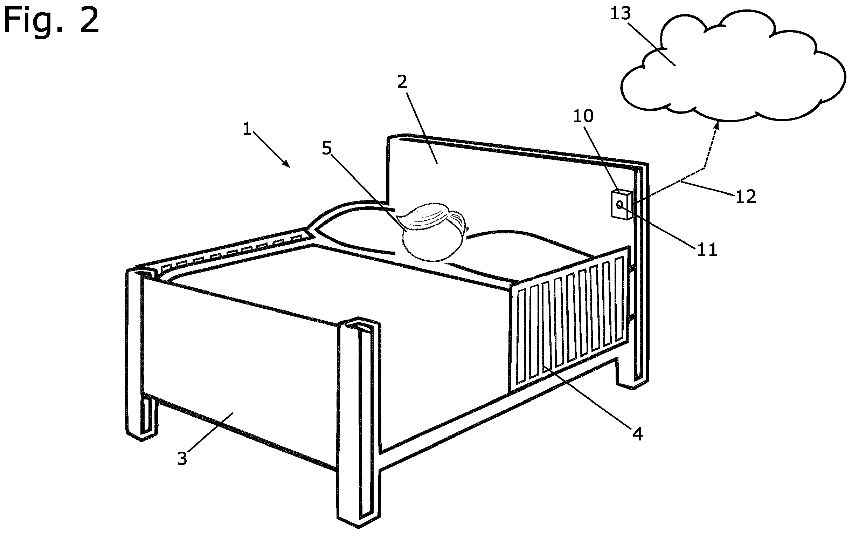

Die Figuren 1 und 2 zeigen in jeweils einer schematischen Darstellung ein Bett 1, insbesondere ein Pflege- oder Klinikbett, mit jeweils einem Kopfteil 2, einem Fußteil 3 und Seitenteilen 4. Im Bett liegend ist schematisch eine Person 5 dargestellt.

Beim Beispiel der Figur 1 erstrecken sich die Seitenteile 4 entlang beider Seiten des Betts 1 über die jeweils gesamte Länge der Bettseite. Beim Beispiel der Figur 2 ist auf einer Seite ein Seitenteil 4 vorhanden, das sich nur über einen Teil der Länge des Betts 1 erstreckt. Ein derartiges Seitenteil 4 wird beispielsweise eingesetzt, wenn ein Herausfallen aus dem Bett 1 verhindert werden soll, aber die Möglichkeit, das Bett zu verlassen, dadurch nicht eingeschränkt werden soll.

Es ist bei beiden Ausführungsbeispielen jeweils eine Sensoranordnung 10 am Kopfteil 2 des Betts 1 montiert, die einen Sensor 11 aufweist, der so ausgerichtet ist, dass ein Raumbereich entlang einer Linie oberhalb des Seitenteils 4 auf Reflexionen durch Gegenstände oder Körperteile, die in diesem Bereich eindringen, überwacht wird.

Dazu weist der Sensor 11 einen Sender für ein Signal auf, dessen eventuelle Reflexion von einem ebenfalls im Sensor 11 enthaltenen Empfänger wieder registriert wird. Der Sensor 11 ist dabei geeignet, eine Entfernung zu dem Ort der Reflexion zu messen. Dieses kann beispielsweise über eine gemessene Signallaufzeit erfolgen. Der Sensor 11 kann bevorzugt ein Lidar-Sensor sein oder auch ein Ultraschallsensor. Alternativ ist auch eine lichtbasierte, beispielsweise infrarotlichtbasierte Triangulationsmessung in Reflexionsgeometrie möglich, um den Abstand zum Reflexionsort zu messen.

Neben der Entfernungsbestimmung über z.B. die Laufzeitmessung kann zusätzlich eine Intensitätsmessung des reflektierten Signals erfolgen, die Aufschluss über eine Größe des reflektierenden Objekts zulässt. Dieses gilt insbesondere in Verbindung mit der gemessenen und damit bekannten Entfernung.

In der Sensoranordnung 10 ist eine Steuer- und gegebenenfalls Auswerteschaltung für den Sensor 11 enthalten sowie eine Kommunikationseinrichtung. Die Kommunikationseinrichtung sendet Rohdaten des Sensors 11 und/oder daraus abgeleitete Daten bzw. Informationen über einen Kommunikationskanal 12 zum Beispiel in ein Netzwerk 13. Über das Netzwerk 13 kann eine Meldung der Sensoranordnung 10 an Warneinrichtungen weitergeleitet werden, die Pflege- oder Betreuungspersonal informieren. Die Warneinrichtungen können beispielsweise mobile Endgeräte auf Basis handelsüblicher Smartphones oder auch speziellen Krankenhaus-Pager sein. Alternativ oder zusätzlich kann ein akustisches oder optisches Warnsignal auch unmittelbar von der Sensoranordnung 10 ausgegeben werden.

Die Messung der Entfernung ermöglicht es, zwischen Bewegungen, die relevant im Hinblick auf ein mögliches Überklettern des Seitenteils 4 sind, und solchen, die beispielsweise außerhalb des Betts 1 liegen, zu unterscheiden. Dadurch, dass der Ort der Bewegung erkannt wird, kann die Sensoreinrichtung auch in einer Anordnung gemäß Figur 2 eingesetzt werden. Es kann zwischen einem nicht zulässigen Überklettern im Bereich des Seitenteils 4 und einem zulässigen Verlassen des Betts im Bereich neben dem Seitenteil 4 unterschieden werden.

Die Sensoranordnung 10 kann einfach am Kopfteil 2 positioniert werden und benötigt zur Installation lediglich zum Beispiel eine Federklemme, Schraubklemme, Klettband, doppelseitiges Klebeband oder ähnliches. Insbesondere bei einem drahtlosen Kommunikationskanal 12 ist nur ein geringer oder gar kein Verdrahtungsaufwand erforderlich.

In einem einfachen Fall umfasst eine Auswertung der gemessenen Entfernungen bis zur Reflexion lediglich einen Vergleich mit einem bestimmten Entfernungsbereich. Wird eine Reflexion innerhalb dieses Entfernungsbereichs detektiert, wird das Warnsignal ausgegeben. In einer Weiterbildung kann vorgesehen sein, auch eine Zeitabhängigkeit der der Position des Reflexionsorts zu berücksichtigen, um zwischen eher zufälligen Bewegungen innerhalb des Detektionsbereichs und einem geplanten Überklettern des Seitenteils 4 unterscheiden zu können. Hier kann gegebenenfalls ein Mustervergleich mit Referenzmustern (Entfernungsvariation in Abhängigkeit der Zeit) vorgesehen sein. Auch ist denkbar, den Mustervergleich selbstlernend durchzuführen.

### Bezugszeichen

- 1: Bett
- 2: Kopfteil
- 3: Fußteil
- 4: Seitenteil
- 5: Person

- 10: Sensoranordnung
- 11: Sensor
- 12: Kommunikationskanal
- 13: Netzwerk

## Patentansprüche

1. Vorrichtung zum Erfassen einer Bewegung einer Person im Bereich eines Seitenteils (4) eines Betts (1), insbesondere eines Pflege- oder Klinikbetts,
**dadurch gekennzeichnet, dass**
am Bett (1) oberhalb des Seitenteils (4) ein Sensor (11) angeordnet ist, der eine Entfernung zu einem Gegenstand oder Körperteil misst, der oder das sich entlang einer Linie oberhalb des Seitenteils (4) befindet.

2. Vorrichtung nach Anspruch 1, bei der der Sensor (11) ein Lidar-Sensor ist.

3. Vorrichtung nach Anspruch 1, bei der der Sensor (11) ein Ultraschall-Sensor ist.

4. Vorrichtung nach einem der Ansprüche 1 bis 3, bei der der Sensor (11) einen Sender und einen Empfänger für ein Signal in Reflexionsgeometrie umfasst.

5. Vorrichtung nach Anspruch 4, bei der eine Laufzeitmessung zwischen einem Senden und einem Empfangen des Signals zur Entfernungsmessung vorgesehen ist.

6. Vorrichtung nach einem der Ansprüche 1 bis 5, bei der der Sensor (11) an einem Kopfteil des Betts (1) montiert ist.

7. Vorrichtung nach einem der Ansprüche 1 bis 6, bei der der Sensor (11) in einer Sensoranordnung (10) angeordnet ist, die eine Ansteuerschaltung für den Sensor (11), eine Auswerteschaltung für den Sensor (11) und/oder eine Kommunikationseinrichtung aufweist.
